# DEMANDE DE BREVET EUROPEEN

(11) **EP 1 329 193 A2**
(43) Date de publication de la demande: **23.07.2003**
(21) Numéro de dépôt: 03290585.3
(22) Date de dépôt: 12.01.1999
(51) Int. Cl.: A61B 8/10

(54) **Procédé d'exploration et de visualisation de tissus d'origine humaine ou animale à partir d'une sonde ultrasonore à haute fréquence**

(30) Priorité: 12.01.1998 FR 9800209
(62) Demande divisionnaire de: 99900521.8
(71) Demandeur: CENTRE NATIONAL DE LA RECHERCHE SCIENTIFIQUE (CNRS), 75794 Paris Cedex 16 (FR)
(72) Inventeur: La désignation de l'inventeur n'a pas encore été déposée
(74) Mandataire: Peaucelle, Chantal

(57) **Abrégé**

L'invention concerne une application d'une sonde ultrasonore pour l'exploration et la visualisation en 2D ou 3D de tissus d'origine humaine et animale, caractérisée en ce qu'il s'agit d'une sonde à focalisation dynamique, capable de générer des faisceaux d'ondes convergentes ultrasonores dans une plage de fréquence nominale variant de 30 à 100 MHz avec une large bande passante adaptée aux fréquences réfléchies par le tissu exploré.

## Description

La présente invention est relative à un procédé d'exploration et de visualisation, mettant en oeuvre des techniques d'échographie ultrasonore, de structures de tissus d'origine humaine ou animale tels que notamment des globes oculaires et plus particulièrement au niveau du segment postérieur (la cavité vitréenne, la paroi postérieure du globe tapissée par la choroïde et la rétine, la macula), celles du segment antérieur (la cornée, la chambre antérieure, l'iris et le cristallin). Elle vise également un dispositif et une sonde ultrasonore permettant de réaliser cette exploration et cette visualisation en 2D ou 3D.

En imagerie ultrasonore et plus particulièrement en échographie médicale, le choix de la fréquence est imposé par le compromis résolution/profondeur de pénétration. En effet, en raison de l'augmentation de l'atténuation des ondes ultrasonores avec la fréquence, la profondeur de pénétration des ultrasons est d'autant plus importante que la fréquence est basse. Par contre, la résolution des images diminue lorsque la fréquence diminue.

On connaît, notamment par le brevet FR-2 620 327, des procédés d'exploration de structures oculaires, par échographie, utilisant des sondes fonctionnant à basse fréquence de l'ordre de 10 MHz, et focalisées à une profondeur sensiblement égale à la dimension d'un globe oculaire (environ 23 à 25 mm). Ces procédés permettent, d'une part de réaliser des images en coupe avec des résolutions spatiales proches du millimètre au niveau du segment postérieur de l'oeil, et d'autre part de pratiquer un examen très grossier de la globalité du segment antérieur de l'oeil.

L'inconvénient majeur de l'échographie à basse fréquence réside principalement dans la faible résolution (600 à 700 µm) qu'offrent ces basses fréquences, celles-ci ne permettant pas une analyse fine de la rétine et des autres couches de la paroi postérieure de l'oeil (choroïde et sclère) et plus particulièrement au niveau de la région maculaire.

Afin d'affiner la résolution, tant latérale qu'axiale, des procédés d'exploration et de visualisation mettant en oeuvre des sondes ultrasonores à haute fréquence, de l'ordre de 50 à 100 MHz (cf. US-5 551 432 et C.J. PAVLIN, M.D. SHERAR, F.S. FOSTER : Subsurface ultrasound microscopic imaging of the intact eye. Ophtalmology 97 :244,1990), à courte focale (de l'ordre de 4 à 8 mm), ont permis d'explorer avec une résolution de 50 µm des structures du segment antérieur de l'oeil, à des profondeurs de l'ordre de 5 mm, ou des structures de la rétine périphérique très proches du segment antérieur.

En conclusion, il est donc admis que les hautes fréquences apparaissent limitées à l'exploration du segment antérieur et de la rétine périphérique, tandis que l'exploration des structures profondes (segment postérieur) nécessite l'emploi de fréquences beaucoup plus basses, tout en n'offrant que des résolutions spatiales très réduites, quelques centaines de microns.

La présente invention vise à pallier les inconvénients des procédés connus de l'art antérieur, en proposant un procédé d'exploration et de visualisation utilisant une sonde ultrasonore à haute fréquence qui allie à la fois une très bonne résolution spatiale et un champ d'exploration couvrant les segments antérieur et postérieur du globe oculaire.

A cet effet, le procédé d'exploration et de visualisation de tissus d'origine humaine ou animale se caractérise en ce que :
- on positionne une sonde ultrasonore portée par une tête pilotée par l'intermédiaire d'un système de positionnement dans les trois dimensions, notamment commandé par un ordinateur au droit de ladite structure de tissus,
- on commande la sonde de manière à ce qu'elle génère des faisceaux d'ondes convergentes ultrasonores de haute fréquence, ces ondes étant focalisées au niveau d'une zone donnée de structure de tissus,
- on effectue un balayage de la structure de tissus par le système de positionnement piloté par l'ordinateur, en effectuant parallèlement une acquisition, par l'ordinateur, des signaux réfléchis par la structure de tissus,
- on effectue divers traitements de signal sur les données issues du balayage, pour améliorer la restitution des informations et faciliter l'interprétation par le praticien.

Selon une autre caractéristique avantageuse de l'invention, on excite la sonde de manière à ce qu'elle génère des faisceaux d'ondes dont la fréquence nominale est comprise dans la fourchette de 30 à 100 MHz avec une large bande passante, adaptée aux fréquences réfléchies par la structure explorée.

Selon encore une autre caractéristique avantageuse de l'invention, les faisceaux d'ondes sont focalisés selon une distance verticale de pénétration comprise entre 20 et 30 mm.

D'autres caractéristiques et avantages de la présente invention ressortiront de la description faite ci-après, en référence aux dessins annexés qui en illustrent un exemple de réalisation dépourvu de tout caractère limitatif. Sur les figures :
- la figure 1 est une vue synoptique d'un dispositif permettant la mise en oeuvre du procédé objet de l'invention ;
- la figure 2 est une vue illustrant une utilisation du procédé objet de l'invention pour l'exploration du segment postérieur d'un globe oculaire ;
- la figure 3 est une vue illustrant une utilisation du procédé objet de l'invention pour l'exploration du segment antérieur d'un globe oculaire ;
- les figures 4a et 4b illustrent, d'une part une vue de face d'un mode de réalisation de la sonde ultrasonore composée d'un réseau annulaire dont le point de focalisation peut être modifié électroniquement, et d'autre part une vue latérale de cette même sonde dans laquelle on introduit un déphasage à l'émission ou à la réception entre les différents anneaux constituant le réseau ;
- la figure 5 est une vue illustrant une utilisation du procédé objet de l'invention pour l'exploration du segment antérieur d'un globe oculaire, utilisant une sonde à focalisation dynamique ;
- la figure 6 est une vue illustrant une utilisation du procédé objet de l'invention pour l'exploration du segment postérieur d'un globe oculaire, utilisant une sonde à focalisation dynamique ;
- la figure 7 montre une comparaison d'une coupe maculaire, d'un globe humain *in vitro,* obtenue par image histologique macroscopique (partie droite) et par une image issue du procédé objet de l'invention (partie gauche) où P représente des plis rétiniens, R la rétine, S la sclère, V le vitré ;
- la figure 8 est l'image obtenue d'un segment antérieur d'un oeil de lapin, par le procédé objet de l'invention, où C représente la cornée, I l'iris, S la sclère, Cr la face antérieure du cristallin.

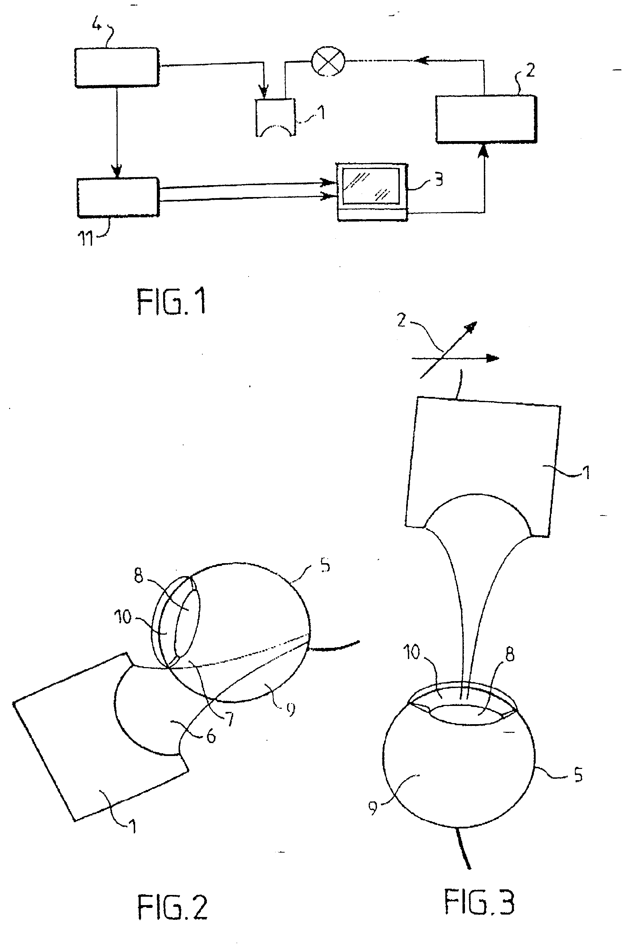

Selon un mode préféré de réalisation du procédé objet de l'invention, dont une installation permettant sa mise en oeuvre est représentée schématiquement sur la figure 1, celui-ci consiste à positionner une sonde ultrasonore 1 montée au sein d'une tête articulée dans les trois dimensions X, Y, Z, l'une au moins d'entre elles pouvant être figée; cette tête étant pilotée par un système d'asservissement 2 de position, commandé par un ordinateur 3, dans une direction notamment perpendiculaire au milieu à explorer.

Cette sonde ultrasonore 1 consiste essentiellement en un transducteur, notamment en PVDF (Polyvinylidènedifluoride), commandé par un émetteur/récepteur 4, afin de générer des faisceaux d'ondes convergentes ultrasonores de large bande, ces ondes pouvant prendre un profil sphérique ou linéaire.

Ainsi, on se reportera à la figure 2, pour une exploration du segment postérieur d'un globe oculaire 5 préalablement inséré dans un milieu de couplage 6, celui-ci n'altérant pas la propagation des ondes, notamment au niveau de la rétine. On utilise une sonde 1 positionnée sur la pars plana 7, pour éviter l'absorption du faisceau ultrasonore par le cristallin 8 (ce cristallin délimitant par ailleurs le segment postérieur 9 du segment antérieur 10 d'un globe oculaire 5). Cette sonde 1 émet des faisceaux d'ondes ultrasonores calibrées dans une plage de fréquence nominale à large bande variant de 30 à 100 MHz, mettant en jeu des longueurs d'onde allant de 50 à 15 µm, focalisées à une distance focale comprise entre 20 et 30 mm et préférentiellement 25 mm, correspondant en fait à une focalisation à une profondeur moyenne d'un globe oculaire.

On obtient par exemple pour une sonde de fréquence nominale 50 MHz, des résolutions latérale et axiale respectivement de 220 et 70 µm à la focale.

Le système de réception aura une bande passante adaptée aux fréquences réfléchies par la structure, ces fréquences étant plus basses que les fréquences émises en raison de l'atténuation du milieu traversé.

Pour une exploration du segment antérieur (cf. figure 3), on utilise cette même sonde 1 et dans les mêmes conditions de commande que précédemment, dans une position décalée sur l'axe vertical (axe Z) d'une distance correspondant en fait à la distance focale précédente.

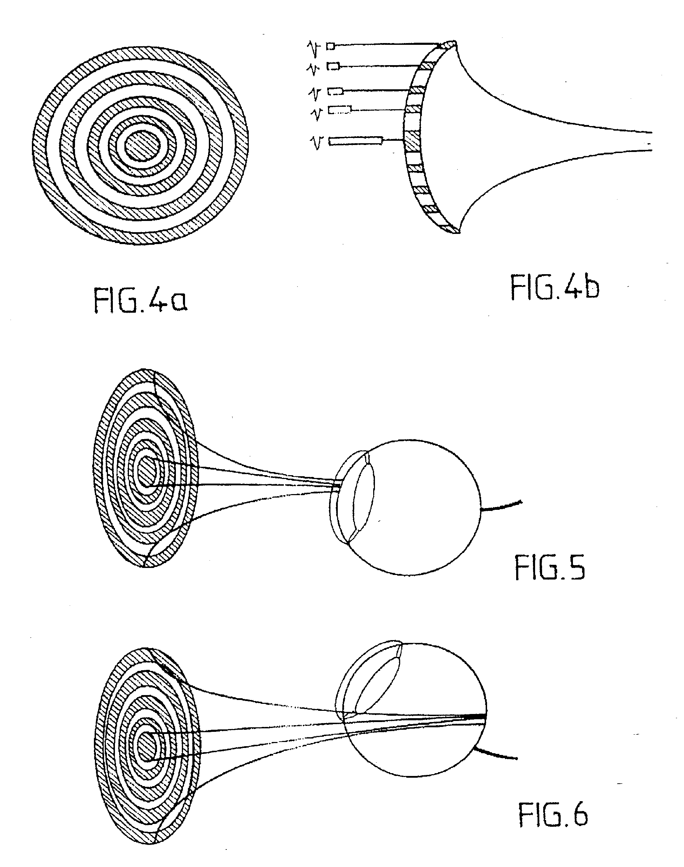

Selon un autre mode de réalisation, la distance focale, notamment selon l'axe de pénétration verticale, n'est pas modifiée par un asservissement mécanique 2 de la position, mais par un dispositif électronique ou numérique pilotant la sonde et permettant de modifier, par une commande judicieuse, la zone de focalisation de la sonde, afin d'obtenir ainsi simultanément une image de bonne résolution du segment antérieur et du segment postérieur de l'oeil. Cette sonde à focalisation dynamique réalisée par un procédé de commande électronique ou numérique, est composée d'une sonde multi-éléments, à symétrie circulaire, composée de plusieurs transducteurs annulaires concentriques régulièrement espacés sur une surface plane ou à concacité sphérique (se reporter à la figure 4a). Ces transducteurs sont indépendants les uns des autres et sont commandés individuellement à l'émission et à la réception par des impulsions décalées dans le temps (se reporter à la figure 4b qui illustre une focalisation dynamique obtenue en introduisant un déphasage - retard temporel - à l'émission entre les différents anneaux).

A l'émission, le front d'onde généré est convergent et sa courbure est modifiée en fonction de la distance entre la structure explorée et la sonde. Les anneaux périphériques émettent en premier et l'excitation de l'anneau central est la plus retardée. Ainsi, la distance focale le long de l'axe de la sonde peut être variable et est donc déterminée par le déphasage ou retard temporel introduit entre les différents transducteurs. Le même principe de focalisation dynamique est utilisé à la réception : le retard électronique est ajusté à la profondeur des échos qui arrivent à cet instant sur la sonde. C'est ainsi que la profondeur de champ est augmentée sans pour autant dégrader la résolution latérale.

Une chaîne de mesure dont chacun des composants (numériseur 11, ordinateur 3, électronique de commande 2, émetteur/récepteur 4...) la constituant possède une bande passante compatible avec le traitement et l'analyse des signaux en provenance du segment antérieur et/ou des signaux issus du segment postérieur de l'oeil, permet un traitement des signaux rétrodiffusés par la structure explorée. Ainsi, le signal ultrasonore rétrodiffusé est amplifié puis digitalisé à l'aide du numériseur 11, à une fréquence d'échantillonnage donnée (notamment de l'ordre de 400 MHz sur 8 bits).

Ce même ordinateur pilote des moteurs pas à pas ou à courant continu afin d'assurer le déplacement de la sonde et le balayage des faisceaux ultrasonores au-dessus de l'échantillon selon un pas déterminé en X et en Y pour permettre un autre point de mesure ou selon un pas R, Ω en utilisant une tête support de sonde permettant un balayage arciforme.

Pour des mesures et des explorations in vivo, il est nécessaire, afin de s'affranchir des déplacements parasites de l'oeil dans son orbite, de traiter le signal en temps réel et de disposer d'un système de déplacement de la sonde extrêmement rapide et précis.

Selon une autre caractéristique, l'ordinateur est équipé d'un module de traitement de l'image et du signal radio fréquence. Ce module comprend des logiciels programmés permettant les deux approches quantitatives de biométrie 2D et/ou 3D et de caractérisation tissulaire.

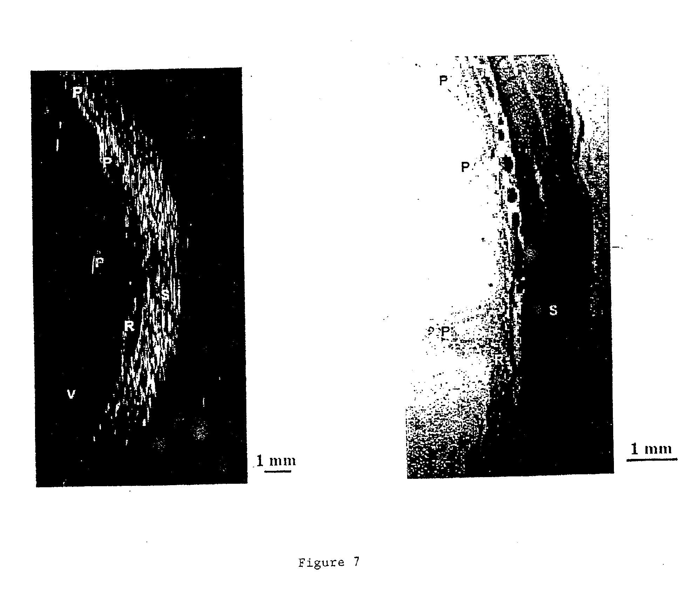

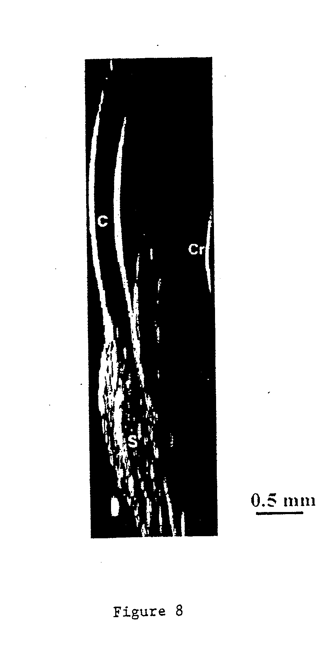

Le signal échographique peut être représenté en temps réel sous la forme d'une ligne A-scan ou sous la forme d'une image 2D de type B-scan. Les images B-scan peuvent visualiser des coupes dans les différents plans parallèles à la direction de propagation des ultrasons (cf. figures 7 et 8). Une image 2D de type C-scan peut également être calculée afin de visualiser des coupes dans le plan perpendiculaire à la direction de propagation des ultrasons. Le C-scan peut représenter des coupes situées à des profondeurs différentes de la totalité du globe oculaire.

Le calcul et la reconstruction d'une image 3D peuvent être obtenus à l'aide de fonctions mathématiques programmées spécifiques aux données ultrasonores à traiter.

Ainsi, connaissant la vitesse de propagation des ultrasons dans les structures explorées, il est possible de déterminer des caractéristiques morphologiques de ces structures, notamment leur épaisseur et/ou leur volume.

Les logiciels de traitement du signal radio fréquence permettent d'analyser en fréquence les signaux rétrodiffusés numérisés et enregistrés afin de calculer des paramètres ultrasonores quantitatifs en vue de la caractérisation tissulaire. Ces paramètres sont notamment le coefficient d'atténuation en dB/cm.MHz (décibels/cm.MégaHertz), le coefficient intégral d'atténuation en dB/cm, le coefficient de rétrodiffusion en dB/cm. MHz et le coefficient intégral de rétrodiffusion en dB/cm.

Ces paramètres peuvent être estimés localement et leurs valeurs peuvent être représentées sous la forme d'images (images paramétriques).

Il est évidemment possible d'ajouter d'autres algorithmes de traitement du signal radio fréquence et de l'image qui pourraient apporter des informations quantitatives morphologiques et/ou tissulaires susceptibles de caractériser les structures de l'oeil.

Les images obtenues par ce procédé d'exploration, au niveau d'un globe oculaire, aussi bien dans la région du segment antérieur et du segment postérieur, possèdent une résolution qui est améliorée d'un facteur d'au moins deux à trois fois par rapport à celle obtenue avec les échographes traditionnels et ne sont pas limitées par la transparence des milieux explorés comme notamment avec les moyens optiques d'exploration traditionnels (biomicroscopie, angiograhie) dont la qualité peut être affectée par la présence de cataracte et d'hémorragies.

A titre d'exemple, la figure 7 illustre les similitudes entre une image histologique et une image échographique de la macula d'un oeil humain *(in vitro*), et la figure 8 illustre une image d'un segment antérieur d'un oeil de lapin.

Le procédé et le dispositif qui permet sa mise en oeuvre, tels que décrits précédemment, ne sont pas limités à des applications en ophtalmologie, mais ils peuvent trouver des applications en gynécologie et obstétrique, en gastroentérologie, et dans le domaine des examens cardio-vasculaires et par coelioscopie, ou en dermatologie et plus généralement dans tout milieu qui réfléchit un signal exploitable.

Particulièrement, dans le domaine de la dermatologie, il est possible, grâce au procédé d'exploration et de visualisation objet de l'invention, d'explorer les différentes épaisseurs de tissus formant la peau. Ainsi, il est possible par exemple, en effectuant un traitement du signal, de qualifier le degré d'hydratation de la peau, d'apprécier la cicatrisation d'un tissu, de localiser une tumeur et de l'explorer, et enfin plus généralement, d'avoir accès à un grand nombre de pathologies couramment rencontrées en dermatologie.

Le point ou la zone de focalisation du faisceau d'ondes sera réglé dans une fourchette allant de quelques dizièmes de millimètres à plusieurs millimètres et la gamme d'ondes utilisée sera comprise entre 30 et 100 MHz.

Il demeure bien entendu que la présente invention n'est pas limitée aux exemples de réalisation décrits et représentés ci-dessus, mais qu'elle en englobe toutes les variantes.

## Revendications

1. Application d'une sonde ultrasonore pour l'exploration et la visualisation en 2D ou 3D de tissus d'origine humaine et animale,
**caractérisée en ce qu'**il s'agit d'une sonde à focalisation dynamique, capable de générer des faisceaux d'ondes convergentes ultrasonores dans une plage de fréquence nominale variant de 30 à 100 MHz avec une large bande passante adaptée aux fréquences réfléchies par le tissu exploré.

2. Application selon la revendication 1, **caractérisée en ce que** ladite sonde est une sonde multi-éléments, à symétrie circulaire, composée de plusieurs transducteurs annulaires concentriques, régulièrement espacés sur une surface plane ou à concavité sphérique, indépendants les uns des autres, commandés individuellement à l'émission et à la réception par des impulsions décalées dans le temps.

3. Application selon la revendication ou 2, **caractérisée en ce que** la distance focale de la sonde est modifiée par un dispositif électronique.

4. Application selon la revendication 1 ou 2, **caractérisée en ce que** la distance focale de la sonde est modifiée par un dispositif numérique.

5. Application selon l'une quelconque des revendications 1 à 4, pour obtenir simultanément une image de bonne résolution du segment antérieur et du segment postérieur de l'oeil, la distance focale étant ajustée à 4 à 8 mm pour examiner le segment antérieur et de 20 à 30 mm pour examiner le segment postérieur.

6. Application selon l'une quelconque des revendications 1 à 4, en gynécologie et obstétrique, en gastroentérologie, pour des examens cardio-vasculaires ou par coelioscopie, en dermatologie.
